# EUROPEAN PATENT APPLICATION

(11) **EP 4 628 131 A1**
(43) Date of publication of application: **08.10.2025**
(21) Application number: 23896795.4
(22) Date of filing: 28.11.2023
(51) Int. Cl.: A61M 16/00

(54) **FLOW ADJUSTING METHOD FOR VENTILATION THERAPY DEVICE AND VENTILATION THERAPY DEVICE**

(30) Priority: 02.12.2022 CN 202211538546
(71) Applicant: BMC (Tianjin) Medical Co., Ltd., Tianjin 301700 (CN)
(72) Inventor: ZHANG, Lixin, Tianjin 301700 (CN); ZHANG, Anjun, Tianjin 301700 (CN); LAN, Guanglei, Tianjin 301700 (CN); WANG, Qingsong, Tianjin 301700 (CN); ZHUANG, Zhi, Tianjin 301700 (CN)
(74) Representative: Grünecker Patent- und Rechtsanwälte PartG mbB
(86) International application number: PCT/CN2023/134824
(87) International publication number: WO 2024/114647

(57) **Abstract**

The embodiments of the present invention provide a flow adjusting method for a ventilation therapy device and a ventilation therapy device. The method includes: measuring current respiratory parameters of a user and determining a current respiratory state of the user based on the respiratory parameters; outputting at a flow output value according to the current respiratory state of the user; re-obtaining the respiratory parameters of the user after an output valve is controlled according to the flow output value and adjusting a flow value of the output valve based on changes in the respiratory parameters; and adjusting an output airflow flow value of the device in real time by monitoring the respiratory state of the user. Different ventilator output operation schemes are specified for respiratory demands of different users, thus solving the problems in the prior art of too uniform results adjusted by a preset algorithm of the device and poor support for the differentiated needs of different users.

## Description

The present application claims the priority to Chinese Patent Application No. 202211538546.3, filed with the China National Intellectual Property Administration on December 02, 2022 and entitled "FLOW ADJUSTING METHOD FOR VENTILATION THERAPY DEVICE AND VENTILATION THERAPY DEVICE", which is incorporated herein by reference in its entirety.

### Technical Field

The present invention relates to the technical field of medical devices, and in particular to a flow adjusting method for a ventilation therapy device, a ventilation therapy device, an electronic device and a readable storage medium.

### Background Art

A ventilator is a widely used medical auxiliary device that plays an important role in clinical applications for the treatment of respiratory diseases, anaesthesia and respiratory management during major surgery, respiratory support therapy, emergency resuscitation, etc. To meet the needs of different users with different physical conditions and application scenarios, the ventilator is required to have an adaptive means for adjusting the output airflow rate.

In the related art, an automatic airflow (Autoflow) mode is used to provide service support for a ventilation therapy device used by patients. A flow sensor inside the device measures the user's peak respiratory flow rate, and a specific algorithm is used to adaptively adjust the output airflow rate of the device.

However, in existing technical solutions, specific values adjusted for an output airflow of the ventilator are solely defined by the device according to a preset algorithm. The adjustment results from the preset algorithm are too uniform, resulting in providing poor support for the varying needs of users with different physical conditions.

### Summary

Embodiments of the present invention provide a flow adjusting method for a ventilation therapy device, a ventilation therapy device, an electronic device and a readable storage medium, in order to solve the problem in the prior art of the adjustment results of the airflow output by a ventilator are too uniform and failing to accommodate the varied needs of different individuals.

**In** a first aspect, the embodiments of the present invention provide a flow adjusting method for a ventilation therapy device, the flow adjusting method comprising:
measuring current respiratory parameters of a user and obtaining an input supported flow range, the respiratory parameters comprising a peak respiratory flow rate and a respiratory rate of the user;
determining a current respiratory state of the user based on the respiratory rate and the peak flow rate, the supported flow range being a flow range that is determined based on the current respiratory state of the user and meets respiratory demands of the user;
determining a flow output value corresponding to the current respiratory state of the user based on the supported flow range and the current respiratory state of the user; and
controlling an output valve of the ventilation therapy device to output at the corresponding flow output value.

**In** a second aspect, the embodiments of the present invention provide a ventilation therapy device, comprising:
a device body, a breathing circuit, a user interface, an output valve, and a control module, wherein
the device body is configured to output gas at a preset pressure and a preset flow rate through an output end, the breathing circuit comprises a first end and a second end that are in communication with each other, and the first end of the breathing circuit is in communication with the output end;
the second end of the breathing circuit is connected to the user interface, and the user interface is configured to be worn at the nasal cavity of a user; when the user interface is worn at the nasal cavity of the user, an exhalation gap is provided between the user interface and the nasal cavity of the user; and
the control module is configured to: measure current respiratory parameters of the user, the respiratory parameters comprising a peak respiratory flow rate of the user and a time interval between two consecutive peak flow rates; calculate a respiratory rate based on the time interval between two consecutive peak flow rates, and determine a current respiratory state of the user based on the respiratory rate and the peak flow rate; determine a flow output value corresponding to the current respiratory state of the user; and after controlling the output valve based on the flow output value, obtain updated respiratory parameters of the user and adjust a flow value of the output valve.

In a third aspect, the embodiments of the present invention provide an electronic device, comprising: a processor; and
a memory configured to store instructions executable by the processor,
wherein the processor is configured to execute the instructions to implement the method.

In a fourth aspect, the embodiments of the present invention provide a readable storage medium. Instructions in the readable storage medium, when executed by a processor of an electronic device, cause the electronic device to carry out the method.

In an embodiment of the present invention, by means of the ventilation therapy device, current respiratory parameters of a user are measured first, and a supported flow range input to the device is obtained, the respiratory parameters comprising a peak respiratory flow rate of the user and a respiratory rate of the user; a current respiratory state of the user is determined based on both the respiratory rate and the peak flow rate, and then outputting is performed at a corresponding flow output value according to the current respiratory state of the user; and an output airflow flow value of the device is adjusted by monitoring the respiratory state of the user. Different ventilator output operation schemes are specified for respiratory demands of different users, thus solving the problems in the prior art of too uniform results adjusted by a preset algorithm of the device and poor support for the differentiated needs of different users.

The above-mentioned description is merely a summary of the technical solutions of the present invention. In order to more clearly understand the technical means of the present invention so that the implementation can be carried out according to the content of the description, and in order to make the above-mentioned and other objects, features and advantages of the present invention be more comprehensible, the particular embodiments of the present invention will be illustrated below.

### Brief Description of the Drawings

By reading the detailed description of the preferred embodiments below, various other advantages and benefits will become clear and apparent to those skilled in the art. The accompanying drawings are merely for the purpose of illustrating the preferred embodiments, and are not considered to be a limitation to the present invention. In addition, throughout the drawings, the same reference symbols are used to represent the same components. In the figures:
Fig. 1 is a simplified flow chart of steps for implementing a flow adjusting method for a ventilation therapy device according to an embodiment of the present invention;
Fig. 2 is a graph showing the changes in respiratory airflow of a user according to an embodiment of the present invention;
Fig. 3 is a complete flow chart of steps of a flow adjusting method for a ventilation therapy device according to an embodiment of the present invention;
Fig. 4 is another complete flow chart of steps of a flow adjusting method for a ventilation therapy device according to an embodiment of the present invention;
Fig. 5 is another complete flow chart of steps of a flow adjusting method for a ventilation therapy device according to an embodiment of the present invention;
Fig. 6 is another complete flow chart of steps of a flow adjusting method for a ventilation therapy device according to an embodiment of the present invention;
Fig. 7 is another complete flow chart of steps of a flow adjusting method for a ventilation therapy device according to an embodiment of the present invention;
Fig. 8 is another complete flow chart of steps of a flow adjusting method for a ventilation therapy device according to an embodiment of the present invention;
Fig. 9 is another complete flow chart of steps of a flow adjusting method for a ventilation therapy device according to an embodiment of the present invention;
Fig. 10 is a schematic diagram showing a composition relationship of functional components of a ventilation therapy device according to an embodiment of the present invention;
Fig. 11 is a relationship diagram of functional components of an electronic device according to an embodiment of the present invention; and
Fig. 12 is a composition diagram of a readable storage medium according to an embodiment of the present invention.

### Detailed Description of Embodiments

Exemplary embodiments of the present invention will be described in more detail below with reference to the accompanying drawings. Although the exemplary embodiments according to the present invention are shown in the accompanying drawings, it should be understood that the present invention can be implemented in various forms and should not be limited by the embodiments set forth herein. In contrast, these embodiments are provided for a more thorough understanding of the present invention, and for fully conveying the scope of the present invention to those skilled in the art.

A flow adjusting method for a ventilation therapy device provided by the present invention will be described in detail below by way of some specific embodiments.

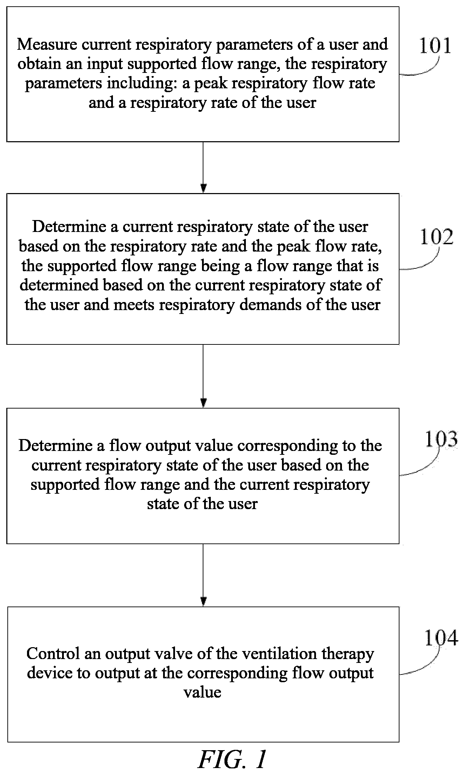

Fig. 1 is a simplified flow chart of steps for implementing a flow adjusting method for a ventilation therapy device according to an embodiment of the present invention. The method may include the following steps.

In step 101, current respiratory parameters of a user are measured and an input supported flow range is obtained. The respiratory parameters include a peak respiratory flow rate and a respiratory rate of the user.

In an embodiment of the present invention, a flow adjusting method for a ventilation therapy device is provided, which can adaptively and dynamically regulate an operating mode according to a physical condition of a user. First, a ventilation connection is established between the ventilation therapy device and the user by means of a breathing circuit. The user receives airflow output from the device by wearing, at the nasal cavity, an interface at an end of the circuit. Moreover, a device end can also reversely receive the breath changes during the user's breathing through the circuit. In the embodiment of the present invention, a control module of the device is provided with a flow sensor capable of monitoring changes in the respiratory airflow of the user in real time.

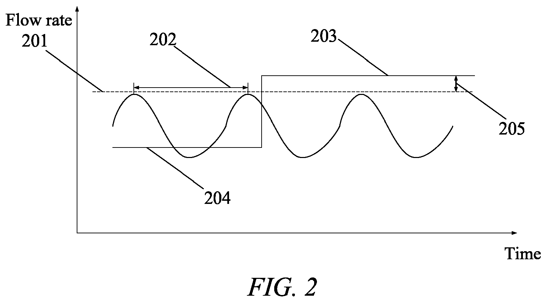

Referring to Fig. 2, Fig. 2 is a graph showing changes in respiratory airflow of a user according to an embodiment of the present invention. As shown in Fig. 2, the graph includes: a peak flow rate 201, a peak-to-peak time interval line 202, a standard output value reference line 203, a non-standard output value reference line 204, and a redundant flow value line 205. The graph has two-dimensional reference coordinates, where the horizontal axis represents time and the vertical axis represents specific airflow flow values. The measured respiratory airflow flow values of the user vary over time to generate a corresponding monitoring curve. During this process, the ventilation therapy device mainly obtains two respiratory parameters: the peak respiratory flow rate of the user and the time interval between two consecutive peak flow rates. The respiratory rate of the user can be determined by calculating the number of occurrences of the time intervals between peak flow rates within a unit of time and rounding the result. For example, if there are 19 respiratory flow rate peaks in total within a one-min monitoring period, and 18 peak-to-peak time interval lines 202 exist between the corresponding peaks, then the current respiratory rate of the user is 18 breaths per minute (min).

In practical applications, the measured airflow flow can be obtained through a measured airflow flow rate and geometrical parameters of the circuit, where the flow rate is directly proportional to the sectional area (i.e., the cross-sectional area) of a tube and the flow rate, expressed by a calculation formula: Q = S • V, where Q represents the obtained airflow rate, in cubic meters per hour (m³/h), S represents the area of the circuit for connecting an airflow source and a monitoring unit, in square meters (m²), and V represents an average velocity of a fluid, in meters per second (m/s).

Based on the measured respiratory parameters, the current respiratory state of the user can be further obtained, different airflow output schemes corresponding to the different respiratory states are then matched, and an output airflow flow value of the device can be adaptively adjusted by controlling the output valve of the device to meet the different respiratory needs of different users.

In step 102, a current respiratory state of the user is determined based on the respiratory rate and the peak flow rate. The supported flow range is a flow range that is determined based on the current respiratory state of the user and meets respiratory demands of the user.

Referring to Fig. 2, the peak flow rate line 201 indicates a peak respiratory airflow of the user, i.e., the maximum airflow through the nasal cavity during a single breathing action of the user, over a steady-state measurement period, and the peak-to-peak time interval line 202 indicates a time interval between the peak flow rates of two consecutive breaths on a measurement curve. In the embodiments of the present invention, it is assumed that the time intervals between the peak flow rates of every two consecutive breaths of the user in a steady state are approximately equal, and a target respiratory rate can thus be obtained by taking the reciprocal of a single time interval. The respiratory rate is used to represent the total number of complete breaths taken by the user within a fixed time period, for example, measuring the target respiratory rate per minute, in breaths per minute.

Further, the current respiratory state of the user is determined based on both the respiratory rate and the peak flow rate. In an embodiment of the present invention, medical personnel may set different respiratory parameter determination thresholds for the ventilation therapy device. The current respiratory state of the user is determined by comparing the measured respiratory parameters with preset thresholds.

Optionally, step 102 may specifically include the following sub-steps.

**In** sub-step 1021, the respiratory rate is compared with a first rate threshold and a second rate threshold, respectively, and the peak flow rate is compared with a first flow rate threshold and a second flow rate threshold, respectively, to obtain comparison results.

The current respiratory state of the user is determined based on the respiratory rate obtained in step 102 and the peak flow rate; and the respiratory state of the user is determined by comparing the respiratory rate with the preset rate thresholds, and the peak flow rate with the flow rate thresholds.

The first rate threshold is greater than the second rate threshold, and the first flow rate threshold is less than the second flow rate threshold.

**In** an optional implementation provided by an embodiment of the present invention, the first rate threshold set for the determination of the respiratory rate is 20 breaths per minute (20 Bpm) and the second rate threshold is 12 breaths per minute (12 Bpm). Likewise, the first flow rate threshold set for the determination of the peak flow rate is 20 litres per minute (20 L/min) and the second flow rate threshold is 35 litres per minute (35 L/min). The above thresholds may be adaptively adjusted according to actual situations for different criteria used by different medical personnel, and are not particularly limited in the embodiments of the present invention.

**In** sub-step 1022, the current respiratory state of the user is determined based on the comparison results.

As can be seen from the above, the first rate threshold and the second rate threshold can define three threshold intervals: the respiratory rates less than 12 Bpm, the respiratory rates greater than 12 Bpm and less than 20 Bpm, and the respiratory rates greater than 20 Bpm. Similarly, the first flow rate threshold and the second flow rate threshold can also define three threshold intervals: the peak flow rates less than 20 L/min, the peak flow rates greater than 20 L/min and less than 35 L/min, and the peak flow rates greater than 35 L/min. Two obtained respiratory parameters are compared with the thresholds to determine which threshold intervals the current respiratory parameters fall into, thereby determining the current respiratory state of the user.

**In** step 103, a flow output value corresponding to the current respiratory state of the user is determined based on the supported flow range and the current respiratory state of the user.

For different respiratory states, the medical personnel may preset output schemes corresponding to different states for the ventilation therapy device. **In** practical applications, the medical personnel adopt different respiratory adaptation schemes for users with varying respiratory needs. **In** a flow control method for the ventilation therapy device according to the embodiments of the present invention, an output value corresponding to a target output valve flow rate ultimately controlled by the ventilation therapy device mainly includes: a baseline flow value, a preset supported flow value and a preset redundant flow value. The baseline flow value represents a baseline flow rate that satisfies the current respiratory state of the user; **the preset** supported flow value is a respiratory scheme flow value set by the medical personnel for the user to meet respiratory needs, a specific value of which falls within an obtained supported flow range, is determined according to the detected respiratory state and may be adaptively adjusted by the medical personnel for different respiratory demands; and the redundant flow value is a safety value set by the medical personnel for the ventilation therapy device. **In** practical applications, there may be a slight fluctuation in the peak respiratory flow rate of the user, and the safety value is set to ensure that the respiratory demands of the user can be continuously met within the slight fluctuation range.

Specifically, with reference to Fig. 2, in the graph showing changes in respiratory airflow shown in Fig. 2, the horizontal position of the non-standard output value reference line 204 is significantly lower than the peak respiratory flow rate of the user and cannot meet the basic respiratory demands of the user in practical applications. **In** contrast, the horizontal position of the standard output value reference line 203 is higher than the peak respiratory flow rate, with a preset redundant flow value added to the peak flow rate 201. As shown in Fig. 2, the redundant flow value line 205 exists between the standard output value reference line 203 and the peak flow rate 201.

For example, in an optional implementation provided by an embodiment of the present invention, the corresponding flow output value currently obtained from the measured respiratory parameters is composed of a baseline flow value, a preset supported flow value and a preset redundant flow value. The current peak respiratory flow rate of the user measured at the device end is 20 L and the preset supported flow value set by the medical personnel for the respiratory demands of the user is 30 L; therefore, the flow output value ultimately set for the output valve under the control of the ventilation therapy device is: the peak respiratory flow rate of 20 L + the preset supported flow value of 30 L + the preset redundant flow value of 5 L = 55 L.

**In** step 104, the output valve of the ventilation therapy device is controlled to output at the corresponding flow output value.

The device will output at the set flow output value to meet the respiratory demands of the user. For users with different respiratory states, the respiratory state is matched based on the measured respiratory parameters, and a target flow output value is determined according to the correspondence between preset respiratory states and flow output values, such that the ventilation therapy device can perform output operations in an optimal mode under different usage scenarios and for different users, thereby meeting the differentiated needs of different individuals.

**In** an optional embodiment, the method further includes the following step.

**In** step 105, updated respiratory parameters of the user are obtained.

After controlling the ventilation therapy device to control the output valve to output at a flow output value corresponding to the current respiratory state, subsequent changes in the respiratory state of the user are continuously detected. Specifically, the output valve is controlled to adjust the flow output value again with the changes in the respiratory rate and the peak respiratory flow rate in the above respiratory parameters, to adapt to changes in the state of the user. For example, for a user whose respiratory state tends to be normal, the preset supported flow value may be appropriately reduced; conversely, for a user whose respiratory state is not optimistic, the preset supported flow value needs to be appropriately increased to meet respiratory needs.

**In** the flow adjusting method for a ventilation therapy device according to an embodiment of the present invention, after outputting is performed at the corresponding flow output value matched with the initially detected respiratory state of the user, a total output flow rate of the device is continuously and adaptively adjusted based on respiratory feedback results of the user in response to the flow output value. With the control module as the core, the airflow output value of the device is dynamically adjusted to meet the current respiratory needs of the user at all times.

**In** an optional embodiment, the method further includes the following step.

**In** step 106, a flow value of the output valve is adjusted based on the updated respiratory parameters.

**In** an embodiment of the present invention, the flow value of the output valve is adjusted according to the updated respiratory parameters. Specifically, a new respiratory rate and peak respiratory flow rate obtained based on the measured respiratory parameters are compared with the previously measured respiratory parameters, and the flow output value is adjusted again by adopting the corresponding output valve control scheme based on the changes in respiratory parameters.

For a user who has accepted a preliminary respiratory output scheme, the feedback of his/her state changes is an important part of the adaptive dynamic adjustment according to the embodiment of the present invention. Synchronous adjustment of respiratory parameter measurement and control is achieved by means of the control module, so as to adapt in real time to changes in the state of the user. For a user whose respiratory state tends to be normal, there is no need to receive an excess preset flow, and real-time adjustment allows the user to match a current optimal output scheme while reducing unnecessary resource consumption. Conversely, for a user whose respiratory state is not optimistic, it is necessary to continue adding the corresponding flow output value in real time to provide an optimized respiratory adaptation scheme.

In summary, in a flow adjusting method for a ventilation therapy device according to the embodiments of the present invention, by means of the ventilation therapy device, current respiratory parameters of a user are measured first, and a supported flow range input to the device is obtained, the respiratory parameters including a peak respiratory flow rate of the user and a respiratory rate of the user; a current respiratory state of the user is determined based on both the respiratory rate and the peak flow rate, and then outputting is performed at a corresponding flow output value according to the current respiratory state of the user; and an output airflow flow value of the device is adjusted by monitoring the respiratory state of the user. Different ventilator output operation schemes are specified for respiratory demands of different users, thus solving the problems in the prior art of too uniform results adjusted by a preset algorithm of the device and poor support for the differentiated needs of different users.

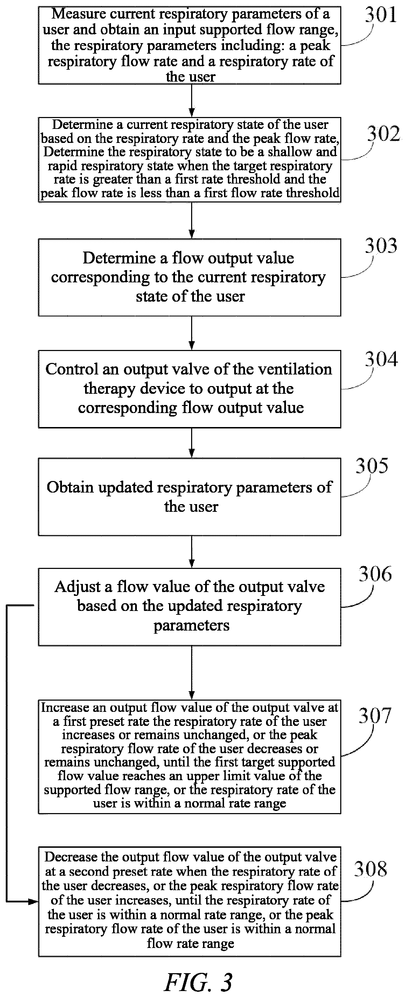

Fig. 3 is a complete flow chart of the steps of a flow adjusting method for a ventilation therapy device according to an embodiment of the present invention. As shown in Fig. 3, the method includes the following steps.

In step 301, current respiratory parameters of a user are measured and an input supported flow range is obtained. The respiratory parameters include a peak respiratory flow rate and a respiratory rate of the user.

The specific implementation of this step may refer to step 101 described above, and will not be described in detail in this embodiment.

**In** step 302, a current respiratory state of the user is determined based on the respiratory rate and the peak flow rate; and when the target respiratory rate is greater than the first rate threshold and the peak flow rate is less than the first flow rate threshold, the respiratory state is determined to be a shallow and rapid respiratory state.

The shallow and rapid respiratory state refers to a respiratory state characterized by a shallower respiratory amplitude and a faster respiratory rhythm, which corresponds to the respiratory parameters including a decreased peak respiratory flow rate and an increased respiratory rate. Specifically, in an optional implementation provided by an embodiment of the present invention, the corresponding respiratory parameters for determining the shallow and rapid respiratory state are set as follows: when the target respiratory rate is greater than the first rate threshold and the peak flow rate is less than the first flow rate threshold, i.e., when the respiratory rate is greater than 20 Bpm and the peak respiratory flow rate is less than 20 L/min, the current respiratory state of the user is determined to be the shallow and rapid respiratory state.

**In** practical applications, the determination criteria for different respiratory states can be adaptively adjusted by the medical personnel according to the actual situations and work experiences, and are not limited in the embodiments of the present invention.

**In** step 303, a flow output value corresponding to the current respiratory state of the user is determined.

The specific implementation of this step may refer to step 103 described above, and will not be described in detail in this embodiment.

Optionally, step 303 may specifically include the following sub-step.

**In** sub-step 3031, a target supported flow value corresponding to the current respiratory state of the user is determined from the supported flow range based on the correspondence between respiratory states and values within the supported flow range, and a flow output value corresponding to the current respiratory state is calculated based on the target supported flow value.

Further, in step 302, after determining that the current respiratory state of the user is the shallow and rapid respiratory state, the flow output value corresponding to the shallow and rapid respiratory state can be matched accordingly. In an embodiment of the present invention, for a user in the shallow and rapid respiratory state, the corresponding target supported flow value is a midpoint of the preset supported flow range. The flow output value corresponding to the current respiratory state is calculated based on the target supported flow value, and the specific flow output value is composed of the sum of the midpoint of the preset supported flow range, the baseline flow value and the preset redundant flow value.

In an embodiment of the present invention, the preset supported flow set by the medical personnel is a value range, with an upper limit value and a lower limit value. For example, in a preferred implementation, for the flow output values corresponding to the user in the shallow and rapid respiratory state, the upper limit value of the preset supported flow value is set to 30 L, and the lower limit value is set to 10 L; then, the midpoint of the preset supported flow range is (10 L+30 L)/2 = 20 L, and the final corresponding flow output value is specifically: the peak respiratory flow rate of 20 L + the midpoint of 20 L of the preset supported flow range + the preset redundant flow value of 5 L = 45 L.

In step 304, the output valve of the ventilation therapy device is controlled to output at the corresponding flow output value.

The specific implementation of this step may refer to step 104 described above, and will not be described in detail in this embodiment.

In step 305, updated respiratory parameters of the user are obtained.

The specific implementation of this step may refer to step 105 described above, and will not be described in detail in this embodiment.

In step 306, a flow value of the output valve is adjusted based on the updated respiratory parameters.

The specific implementation of this step may refer to step 106 described above, and will not be described in detail in this embodiment.

In step 307, when the respiratory rate of the user increases or remains unchanged, or the peak respiratory flow rate of the user decreases or remains unchanged, an output flow value of the output valve at a first preset rate is increased until the first target supported flow value reaches an upper limit value of the supported flow range, or the respiratory rate of the user is within a normal rate range.

After determining the flow output value of the ventilation therapy device as described in step 303, the ventilation therapy device outputs airflow to the user at this value. Within a certain time, when the respiratory parameters of the user change, the device will adjust the flow output value controlled by the output valve correspondingly based on the changed respiratory parameters. If the respiratory rate of the user is higher than or equal to the respiratory rate at a previous moment (i.e., the respiratory rate measured before determining the flow output value), or when the peak respiratory flow rate of the user decreases or remains unchanged, the supported flow of the device correspondingly increases at the first preset rate until the supported flow value reaches the upper limit value of the supported flow range, or until the respiratory rate of the user is within the normal rate range. In an optional implementation provided by an embodiment of the present invention, the first preset rate is specifically set as follows: adjusting the preset supported flow value every 5 minutes at a rate of 10 litres per minute (10 L/min).

In step 308, when the respiratory rate of the user decreases, or the peak respiratory flow rate of the user increases, the output flow value of the output valve is decreased at a second preset rate until the respiratory rate of the user is within the normal rate range, or the peak respiratory flow rate of the user is within a normal flow rate range.

Conversely, when an average respiratory rate of the user is lower than the respiratory rate at a previous moment, or when the peak respiratory flow rate of the user increases, it indicates that the previously set flow output value of the device has taken effect. In this case, the supported flow of the device is decreased at the second preset rate to decrease the output flow value of the output valve, until the respiratory rate of the user is within the normal rate range (the respiratory rate of 12-20 breaths per minute is generally considered normal), or the peak respiratory flow rate of the user is within the normal flow rate range (the peak respiratory flow rate of 20 L-35 L is generally considered normal). In an optional implementation provided by an embodiment of the present invention, the second preset rate is specifically set as follows: adjusting the preset supported flow value every 30 minutes at a rate of 5 litres per minute (5 L/min). The above steps are then repeatedly performed until the measured respiratory rate tends to be normal, at which point the adjustment can be stopped.

In summary, in the flow adjusting method for a ventilation therapy device according to the embodiments of the present invention, by means of the ventilation therapy device, current respiratory parameters of a user are measured first, the respiratory parameters including a peak respiratory flow rate of the user, and a time interval between two consecutive peak flow rates; a target respiratory rate is calculated based on the time interval between two consecutive peak flow rates, a current respiratory state of the user is determined based on both the respiratory rate and the peak flow rate, and then outputting is performed at a corresponding flow output value based on the current respiratory state of the user; the respiratory parameters of the user are re-obtained after an output valve is controlled according to the flow output value, and a flow value of the output valve is adjusted based on changes in the respiratory parameters; and an output airflow flow value of the device is adjusted in real time by monitoring the respiratory state of the user. Different ventilator output operation schemes are specified for respiratory conditions of different users, thus solving the problems in the prior art of too uniform results adjusted by a preset algorithm of the device and poor support for the differentiated needs of different users.

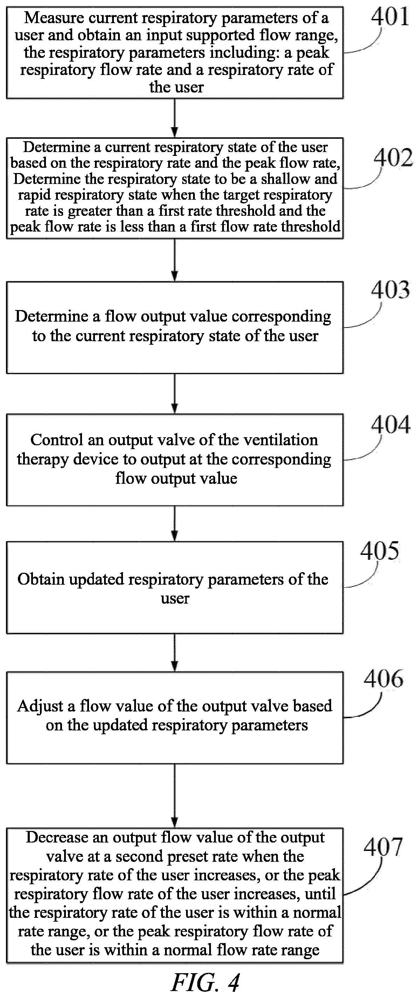

Fig. 4 is another complete flow chart of the steps of a flow adjusting method for a ventilation therapy device according to an embodiment of the present invention. As shown in Fig. 4, the method includes the following steps.

In step 401, current respiratory parameters of a user are measured and an input supported flow range is obtained. The respiratory parameters include a peak respiratory flow rate and a respiratory rate of the user.

The specific implementation of this step may refer to step 101 described above, and will not be described in detail in this embodiment.

In step 402, a current respiratory state of the user is determined based on the respiratory rate and the peak flow rate; and when the target respiratory rate is less than the second rate threshold and the peak flow rate is less than the first flow rate threshold, the respiratory state is determined to be a shallow and slow respiratory state.

The shallow and slow respiratory state refers to a respiratory state characterized by a shallower respiratory amplitude and a slower respiratory rhythm, which corresponds to the respiratory parameters including a decreased peak respiratory flow rate and a decreased respiratory rate. Specifically, in an optional implementation provided by an embodiment of the present invention, the corresponding respiratory parameters for determining the shallow and slow respiratory state are set as follows: when the target respiratory rate is less than the second rate threshold and the peak flow rate is less than the first flow rate threshold, i.e., when the respiratory rate is less than 12 Bpm and the peak respiratory flow rate is less than 20 L/min, the current respiratory state of the user is determined to be the shallow and slow respiratory state.

**In** practical applications, the determination criteria for different respiratory states can be adaptively adjusted by the medical personnel according to the actual situations and work experiences, and are not limited in the embodiments of the present invention.

**In** step 403, a flow output value corresponding to the current respiratory state of the user is determined.

The specific implementation of this step may refer to step 103 described above, and will not be described in detail in this embodiment.

Optionally, step 403 may specifically include the following sub-step.

**In** sub-step 4031, a target supported flow value corresponding to the current respiratory state of the user is determined from the supported flow range based on the correspondence between respiratory states and values within the supported flow range, and a flow output value corresponding to the current respiratory state is calculated based on the target supported flow value.

Further, in step 402, after determining that the current respiratory state of the user is the shallow and slow respiratory state, the flow output value corresponding to the shallow and slow respiratory state can be matched accordingly. **In an** embodiment of the present invention, for a user in the shallow and slow respiratory state, the corresponding target supported flow value is an upper limit value of the preset supported flow range. The flow output value corresponding to the current respiratory state is calculated based on the target supported flow value, and the specific flow output value is composed of the sum of the upper limit value of the **preset** supported flow range, the baseline flow value and the preset redundant flow value.

In an embodiment of the present invention, the preset supported flow set by the medical personnel is a value range, with an upper limit value and a lower limit value. For example, in a preferred implementation, for the flow output values corresponding to the user in the shallow and slow respiratory state, the upper limit value of the preset supported flow value is set to 30 L, and the lower limit value is set to 10 L; then, the final corresponding flow output value is specifically: the peak respiratory flow rate of 20 L + the upper limit value of 30 L of the preset supported flow range + the preset redundant flow value of 5 L = 55 L.

In step 404, the output valve of the ventilation therapy device is controlled to output at the corresponding flow output value.

The specific implementation of this step may refer to step 104 described above, and will not be described in detail in this embodiment.

In step 405, updated respiratory parameters of the user are obtained.

The specific implementation of this step may refer to step 105 described above, and will not be described in detail in this embodiment.

In step 406, a flow value of the output valve is adjusted based on the updated respiratory parameters.

The specific implementation of this step may refer to step 106 described above, and will not be described in detail in this embodiment.

In step 407, when the respiratory rate of the user increases, or the peak respiratory flow rate of the user increases, the output flow value of the output valve is decreased at a second preset rate until the respiratory rate of the user is within the normal rate range, or the peak respiratory flow rate of the user is within a normal flow rate range.

After determining the flow output value of the ventilation therapy device as described in step 403, the ventilation therapy device outputs airflow to the user at this value. Within a certain time, when the respiratory parameters of the user change, the device will adjust the flow output value controlled by the output valve correspondingly based on the changed respiratory parameters. If the respiratory rate of the user increases compared with the respiratory rate at a previous moment, or the peak respiratory flow rate of the user increases, the supported flow of the device is decreased correspondingly at the second preset rate. In a preferred implementation provided by an embodiment of the present invention, the second preset rate is specifically set as follows: adjusting the preset supported flow value every 30 minutes at a rate of 5 litres per minute (10 L/min).

In summary, in the flow adjusting method for a ventilation therapy device according to the embodiments of the present invention, by means of the ventilation therapy device, current respiratory parameters of a user are measured first, the respiratory parameters including a peak respiratory flow rate of the user, and a time interval between two consecutive peak flow rates; a target respiratory rate is calculated based on the time interval between two consecutive peak flow rates, a current respiratory state of the user is determined based on both the respiratory rate and the peak flow rate, and then outputting is performed at a corresponding flow output value based on the current respiratory state of the user; the respiratory parameters of the user are re-obtained after an output valve is controlled according to the flow output value, and a flow value of the output valve is adjusted based on changes in the respiratory parameters; and an output airflow flow value of the device is adjusted in real time by monitoring the respiratory state of the user. Different ventilator output operation schemes are specified for respiratory conditions of different users, thus solving the problems in the prior art of too uniform results adjusted by a preset algorithm of the device and poor support for the differentiated needs of different users.

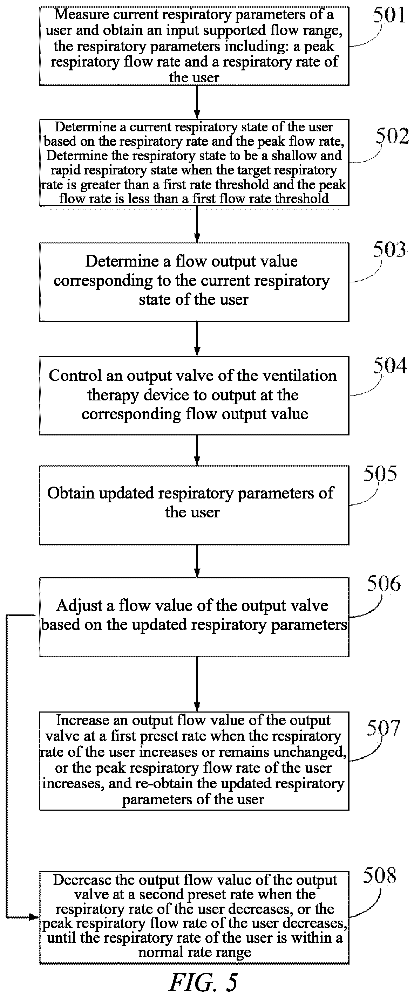

Fig. 5 is another complete flow chart of the steps of a flow adjusting method for a ventilation therapy device according to an embodiment of the present invention. As shown in Fig. 5, the method includes the following steps.

In step 501, current respiratory parameters of a user are measured and an input supported flow range is obtained. The respiratory parameters include a peak respiratory flow rate and a respiratory rate of the user.

The specific implementation of this step may refer to step 101 described above, and will not be described in detail in this embodiment.

In step 502, a current respiratory state of the user is determined based on the respiratory rate and the peak flow rate; and when the target respiratory rate is greater than the first rate threshold and the peak flow rate is greater than the second flow rate threshold, the respiratory state is determined to be a deep and rapid respiratory state.

The deep and rapid respiratory state refers to a respiratory state characterized by an increased respiratory rate and a deeper respiratory amplitude, which corresponds to the respiratory parameters including an increased peak respiratory flow rate and an increased respiratory rate. Specifically, in an optional implementation provided by an embodiment of the present invention, the corresponding respiratory parameters for determining the deep and rapid respiratory state are set as follows: when the target respiratory rate is greater than the first rate threshold and the peak flow rate is greater than the second flow rate threshold, i.e., when the respiratory rate is greater than 20 Bpm and the peak respiratory flow rate is greater than 35 L/min, the current respiratory state of the user is determined to be the deep and rapid respiratory state.

**In** practical applications, the determination criteria for different respiratory states can be adaptively adjusted by the medical personnel according to the actual situations and work experiences, and are not limited in the embodiments of the present invention.

**In** step 503, a flow output value corresponding to the current respiratory state of the user is determined.

The specific implementation of this step may refer to step 103 described above, and will not be described in detail in this embodiment.

Optionally, step 503 may specifically include the following sub-steps.

**In** sub-step 5031, a target supported flow value corresponding to the current respiratory state of the user is determined from the supported flow range based on the correspondence between respiratory states and values within the supported flow range, and a flow output value corresponding to the current respiratory state is calculated based on the target supported flow value.

Further, in step 502, after determining that the current respiratory state of the user is the deep and rapid respiratory state, the flow output value corresponding to the deep and rapid respiratory state can be matched accordingly. **In** an embodiment of the present invention, for a user in the deep and rapid respiratory state, the corresponding target supported flow value is a lower limit value of the preset supported flow range. The flow output value corresponding to the current respiratory **state is** calculated based on the target supported flow value, and the specific flow output value is composed of the sum of the lower limit value of the preset supported flow range, the baseline flow value and the preset redundant flow value.

In an embodiment of the present invention, the preset supported flow set by the medical personnel is a value range, with an upper limit value and a lower limit value. For example, in a preferred implementation, for the flow output values corresponding to the user in the deep and rapid respiratory state, the upper limit value of the preset supported flow value is set to 30 L, and the lower limit value is set to 10 L; then, the final corresponding flow output value is specifically: the peak respiratory flow rate of 20 L + the lower limit value of 10 L of the preset supported flow range + the preset redundant flow value of 5 L = 35 L.

In step 504, the output valve of the ventilation therapy device is controlled to output at the corresponding flow output value.

The specific implementation of this step may refer to step 104 described above, and will not be described in detail in this embodiment.

In step 505, updated respiratory parameters of the user are obtained.

The specific implementation of this step may refer to step 105 described above, and will not be described in detail in this embodiment.

In step 506, a flow value of the output valve is adjusted based on the updated respiratory parameters.

The specific implementation of this step may refer to step 106 described above, and will not be described in detail in this embodiment.

In step 507, when the respiratory rate of the user increases or remains unchanged, or the peak respiratory flow rate of the user increases, an output flow value of the output valve is increased at a first preset rate, and the updated respiratory parameters of the user are re-obtained.

After determining the flow output value of the ventilation therapy device as described in step 503, the ventilation therapy device outputs airflow to the user at this value. Within a certain time, when the respiratory parameters of the user change, the device will adjust the flow output value controlled by the output valve correspondingly based on the changed respiratory parameters. If the respiratory rate of the user is greater than or equal to the respiratory rate at a previous moment, i.e., the respiratory rate measured before determining the flow output value, the supported flow of the device correspondingly increases at the first preset rate. In an optional implementation provided by an embodiment of the present invention, the first preset rate is specifically set as follows: adjusting the preset supported flow value every 5 minutes at a rate of 10 litres per minute (10 L/min).

In step 508, when the respiratory rate of the user decreases, or the peak respiratory flow rate of the user decreases, the output flow value of the output valve is decreased at a second preset rate until the respiratory rate of the user is within a normal rate range.

Conversely, when an average respiratory rate of the user is lower than the respiratory rate at a previous moment, it indicates that the previously set flow output value of the device has taken effect. In this case, the supported flow of the device is decreased at the second preset rate to decrease the output flow value of the output valve. In an optional implementation provided by an embodiment of the present invention, the second preset rate is specifically set as follows: adjusting the preset supported flow value every 30 minutes at a rate of 5 litres per minute (5 L/min). The above steps are then repeatedly performed until the measured respiratory rate tends to be normal, at which point the adjustment can be stopped.

In summary, in the flow adjusting method for a ventilation therapy device according to the embodiments of the present invention, by means of the ventilation therapy device, current respiratory parameters of a user are measured first, the respiratory parameters including a peak respiratory flow rate of the user, and a time interval between two consecutive peak flow rates; a target respiratory rate is calculated based on the time interval between two consecutive peak flow rates, a current respiratory state of the user is determined based on both the respiratory rate and the peak flow rate, and then outputting is performed at a corresponding flow output value based on the current respiratory state of the user; the respiratory parameters of the user are re-obtained after an output valve is controlled according to the flow output value, and a flow value of the output valve is adjusted based on changes in the respiratory parameters; and an output airflow flow value of the device is adjusted in real time by monitoring the respiratory state of the user. Different ventilator output operation schemes are specified for respiratory conditions of different users, thus solving the problems in the prior art of too uniform results adjusted by a preset algorithm of the device and poor support for the differentiated needs of different users.

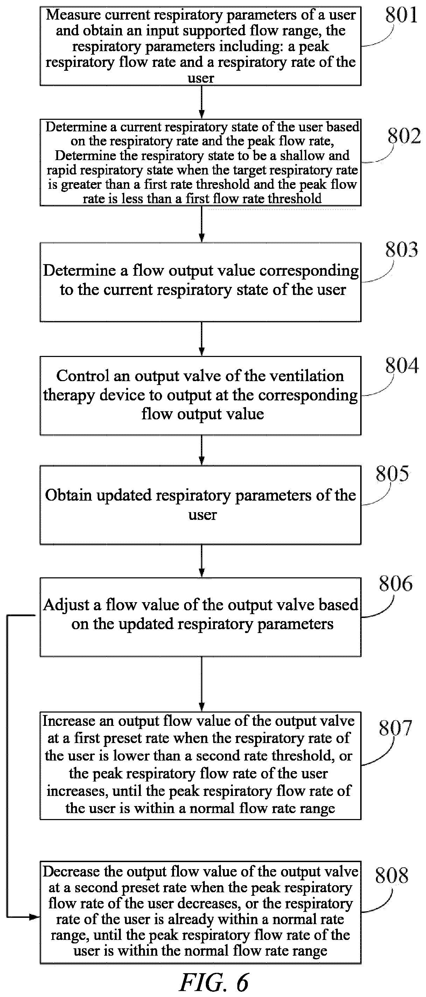

Fig. 6 is another complete flow chart of the steps of a flow adjusting method for a ventilation therapy device according to an embodiment of the present invention. As shown in Fig. 6, the method includes the following steps.

In step 801, current respiratory parameters of a user are measured and an input supported flow range is obtained. The respiratory parameters include a peak respiratory flow rate and a respiratory rate of the user.

The specific implementation of this step may refer to step 101 described above, and will not be described in detail in this embodiment.

In step 802, a current respiratory state of the user is determined based on the respiratory rate and the peak flow rate; and when the target respiratory rate is less than the first rate threshold and greater than the second rate threshold and the peak flow rate is greater than the second flow rate threshold, the respiratory state is determined to be a normal-rate deep respiratory state.

The normal-rate deep respiratory state refers to a respiratory state characterized by a normal respiratory rate and a deeper respiratory amplitude, which corresponds to the respiratory parameters including an increased peak respiratory flow rate and a normal and even respiratory rate. Specifically, in an optional implementation provided by an embodiment of the present invention, the corresponding respiratory parameters for determining the normal-rate deep respiratory state are set as follows: when the target respiratory rate is less than the first rate threshold and greater than the second rate threshold and the peak flow rate is greater than the second flow rate threshold, i.e., when the respiratory rate is between 12 Bpm and 20 Bpm and the peak respiratory flow rate is greater than 35 L/min, the current respiratory state of the user is determined to be the normal-rate deep respiratory state.

In step 803, a flow output value corresponding to the current respiratory state of the user is determined.

The specific implementation of this step may refer to step 103 described above, and will not be described in detail in this embodiment.

Optionally, step 803 may specifically include the following sub-step.

In sub-step 8031, a target supported flow value corresponding to the current respiratory state of the user is determined from the supported flow range based on the correspondence between respiratory states and values within the supported flow range, and a flow output value corresponding to the current respiratory state is calculated based on the target supported flow value.

Further, in step 802, after determining that the current respiratory state of the user is the normal-rate deep respiratory state, the flow output value corresponding to the normal-rate deep respiratory state can be matched accordingly. In an embodiment of the present invention, for a user in the normal-rate deep respiratory state, the corresponding target supported flow value is a lower limit value of the preset supported flow range. The flow output value corresponding to the current respiratory state is calculated based on the target supported flow value, and the specific flow output value is composed of the sum of the lower limit value of the preset supported flow range, the baseline flow value and the preset redundant flow value.

In an embodiment of the present invention, the preset supported flow set by the medical personnel is a value range, with an upper limit value and a lower limit value. For example, in an optional implementation, for the flow output values corresponding to the user in the normal-rate deep respiratory state, the upper limit value of the preset supported flow value is set to 30 L, and the lower limit value is set to 10 L; then, the final corresponding flow output value is specifically: the peak respiratory flow rate of 20 L + the lower limit value of 10 L of the preset supported flow range + the preset redundant flow value of 5 L = 35 L.

In step 804, the output valve of the ventilation therapy device is controlled to output at the corresponding flow output value.

The specific implementation of this step may refer to step 104 described above, and will not be described in detail in this embodiment.

In step 805, updated respiratory parameters of the user are obtained.

The specific implementation of this step may refer to step 105 described above, and will not be described in detail in this embodiment.

In step 806, a flow value of the output valve is adjusted based on the updated respiratory parameters.

The specific implementation of this step may refer to step 106 described above, and will not be described in detail in this embodiment.

In step 807, when the respiratory rate of the user is lower than a second rate threshold, or the peak respiratory flow rate of the user increases, an output flow value of the output valve is increased at a first preset rate until the peak respiratory flow rate of the user is within a normal flow rate range.

After determining the flow output value of the ventilation therapy device as described in step 803, the ventilation therapy device outputs airflow to the user at this value. Within a certain time, when the respiratory parameters of the user change, the device will adjust the flow output value controlled by the output valve correspondingly based on the changed respiratory parameters. If the respiratory rate of the user increases and is higher than the first rate threshold, the supported flow of the device correspondingly increases at the first preset rate. In a preferred implementation provided by an embodiment of the present invention, the first preset rate is specifically set as follows: adjusting the preset supported flow value every 5 minutes at a rate of 10 litres per minute (10 L/min).

In step 808, when the peak respiratory flow rate of the user decreases, or the respiratory rate of the user is already within a normal rate range, the output flow value of the output valve is decreased at a second preset rate until the peak respiratory flow rate of the user is within the normal flow rate range.

Conversely, when an average respiratory rate of the user is lower than the respiratory rate at a previous moment, it indicates that the previously set flow output value of the device has taken effect. In this case, the supported flow of the device is decreased at the second preset rate to decrease the output flow value of the output valve. In an optional implementation provided by an embodiment of the present invention, the second preset rate is specifically set as follows: adjusting the preset supported flow value every 30 minutes at a rate of 5 litres per minute (5 L/min). The above steps are then repeatedly performed until the measured respiratory rate tends to be normal, at which point the adjustment can be stopped.

In summary, in the flow adjusting method for a ventilation therapy device according to the embodiments of the present invention, by means of the ventilation therapy device, current respiratory parameters of a user are measured first, the respiratory parameters including a peak respiratory flow rate of the user, and a time interval between two consecutive peak flow rates; a target respiratory rate is calculated based on the time interval between two consecutive peak flow rates, a current respiratory state of the user is determined based on both the respiratory rate and the peak flow rate, and then outputting is performed at a corresponding flow output value based on the current respiratory state of the user; the respiratory parameters of the user are re-obtained after an output valve is controlled according to the flow output value, and a flow value of the output valve is adjusted based on changes in the respiratory parameters; and an output airflow flow value of the device is adjusted in real time by monitoring the respiratory state of the user. Different ventilator output operation schemes are specified for respiratory states of different users, thus solving the problems in the prior art of too uniform results adjusted by a preset algorithm of the device and poor support for the differentiated needs of different users.

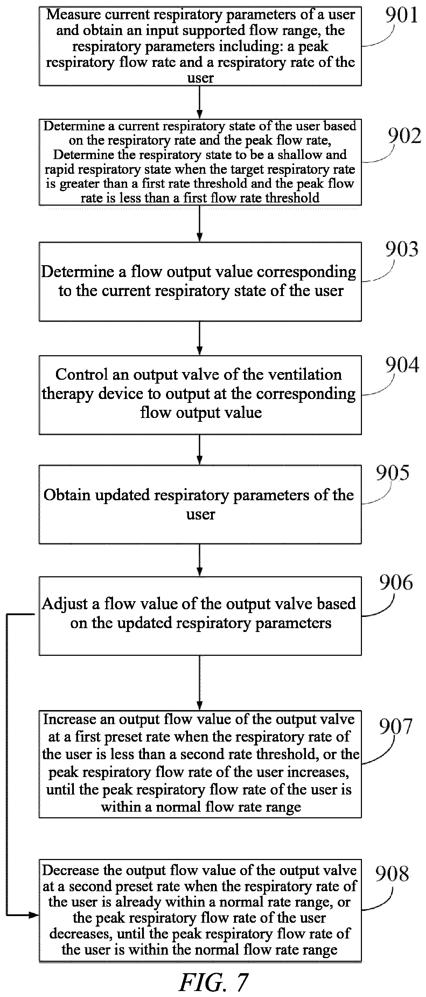

Fig. 7 is another complete flow chart of the steps of a flow adjusting method for a ventilation therapy device according to an embodiment of the present invention. As shown in Fig. 7, the method includes the following steps.

In step 901, current respiratory parameters of a user are measured and an input supported flow range is obtained. The respiratory parameters include a peak respiratory flow rate and a respiratory rate of the user.

The specific implementation of this step may refer to step 101 described above, and will not be described in detail in this embodiment.

In step 902, a current respiratory state of the user is determined based on the respiratory rate and the peak flow rate; and when the target respiratory rate is greater than the second rate threshold and less than the first rate threshold and the peak flow rate is less than the first flow rate threshold, the respiratory state is determined to be a normal-rate shallow respiratory state.

The normal-rate shallow respiratory state refers to a respiratory state characterized by a normal respiratory rate and a shallower respiratory amplitude, which corresponds to the respiratory parameters including a decreased peak respiratory flow rate and a normal and even respiratory rate. Specifically, in an optional implementation provided by an embodiment of the present invention, the corresponding respiratory parameters for determining the normal-rate shallow respiratory state are set as follows: when the target respiratory rate is greater than the second rate threshold and less than the first rate threshold and the peak flow rate is less than the first flow rate threshold, i.e., when the respiratory rate is between 10 Bpm and 20 Bpm and the peak respiratory flow rate is less than 20 L/min, the current respiratory state of the user is determined to be the normal-rate shallow respiratory state.

In practical applications, the determination criteria for different respiratory states can be adaptively adjusted by the medical personnel according to the actual situations and work experiences, and are not limited in the embodiments of the present invention.

In step 903, a flow output value corresponding to the current respiratory state of the user is determined.

The specific implementation of this step may refer to step 103 described above, and will not be described in detail in this embodiment.

Further, in step 902, after determining that the current respiratory state of the user is the normal-rate shallow respiratory state, the flow output value corresponding to the normal-rate shallow respiratory state can be matched accordingly. In an embodiment of the present invention, for a user in the normal-rate shallow respiratory state, the corresponding flow output value is composed of the sum of the lower limit value of the preset supported flow range, the baseline flow value and the preset redundant flow value.

In an embodiment of the present invention, the preset supported flow set by the medical personnel is a value range, with an upper limit value and a lower limit value. For example, in an optional implementation, for the flow output values corresponding to the user in the shallow and rapid respiratory state, the upper limit value of the preset supported flow value is set to 30 L, and the lower limit value is set to 10 L; then, the final corresponding flow output value is specifically: the peak respiratory flow rate of 20 L + the lower limit value of 10 L of the preset supported flow range + the preset redundant flow value of 5 L = 35 L.

In step 904, the output valve of the ventilation therapy device is controlled to output at the corresponding flow output value.

The specific implementation of this step may refer to step 104 described above, and will not be described in detail in this embodiment.

In step 905, updated respiratory parameters of the user are obtained.

After determining the flow output value of the ventilation therapy device as described in step 903, the ventilation therapy device outputs airflow to the user at this value. Within a certain time, when the respiratory parameters of the user change, the device will adjust the flow output value controlled by the output valve correspondingly based on the changed respiratory parameters. If the respiratory rate of the user is greater than the first rate threshold or less than the second rate threshold, the supported flow of the device correspondingly increases at the first preset rate. In an optional implementation provided by an embodiment of the present invention, the first preset rate is specifically set as follows: adjusting the preset supported flow value every 5 minutes at a rate of 10 litres per minute (10 L/min).

In step 906, a flow value of the output valve is adjusted based on the updated respiratory parameters.

Conversely, when an average respiratory rate of the user is lower than the respiratory rate at a previous moment, it indicates that the previously set flow output value of the device has taken effect. In this case, the supported flow of the device is decreased at the second preset rate to decrease the output flow value of the output valve. In an optional implementation provided by an embodiment of the present invention, the second preset rate is specifically set as follows: adjusting the preset supported flow value every 30 minutes at a rate of 5 litres per minute (5 L/min). The above steps are then repeatedly performed until the measured respiratory rate tends to be normal, at which point the adjustment can be stopped.

In step 907, when the respiratory rate of the user is less than a second rate threshold, or the peak respiratory flow rate of the user increases, an output flow value of the output valve is increased at a first preset rate until the peak respiratory flow rate of the user is within a normal flow rate range.

After determining the flow output value of the ventilation therapy device as described in step 903, the ventilation therapy device outputs airflow to the user at this value. Within a certain time, when the respiratory parameters of the user change, the device will adjust the flow output value controlled by the output valve correspondingly based on the changed respiratory parameters. If the respiratory rate of the user is less than the second rate threshold, or when the peak respiratory flow rate of the user increases, the supported flow of the device correspondingly increases at the first preset rate until the peak respiratory flow rate of the user is within the normal flow rate range. In an optional implementation provided by an embodiment of the present invention, the first preset rate is specifically set as follows: adjusting the preset supported flow value every 5 minutes at a rate of 10 litres per minute (10 L/min).

In step 908, when the respiratory rate of the user is already within a normal rate range, or the peak respiratory flow rate of the user decreases, the output flow value of the output valve is decreased at a second preset rate until the peak respiratory flow rate of the user is within the normal flow rate range.

Conversely, when the respiratory rate of the user is already within the normal rate range, or when the peak respiratory flow rate of the user decreases, it indicates that the previously set flow output value of the device has taken effect. In this case, the output flow value of the output valve is decreased at the second preset rate, until the peak respiratory flow rate of the user is within the normal flow rate range. In an optional implementation provided by an embodiment of the present invention, the second preset rate is specifically set as follows: adjusting the preset supported flow value every 30 minutes at a rate of 5 litres per minute (5 L/min). The above steps are then repeatedly performed until the measured respiratory rate tends to be normal, at which point the adjustment can be stopped.

In summary, in the flow adjusting method for a ventilation therapy device according to the embodiments of the present invention, by means of the ventilation therapy device, current respiratory parameters of a user are measured first, the respiratory parameters including a peak respiratory flow rate of the user, and a time interval between two consecutive peak flow rates; a target respiratory rate is calculated based on the time interval between two consecutive peak flow rates, a current respiratory state of the user is determined based on both the respiratory rate and the peak flow rate, and then outputting is performed at a corresponding flow output value based on the current respiratory state of the user; the respiratory parameters of the user are re-obtained after an output valve is controlled according to the flow output value, and a flow value of the output valve is adjusted based on changes in the respiratory parameters; and an output airflow flow value of the device is adjusted in real time by monitoring the respiratory state of the user. Different ventilator output operation schemes are specified for respiratory states of different users, thus solving the problems in the prior art of too uniform results adjusted by a preset algorithm of the device and poor support for the differentiated needs of different users.

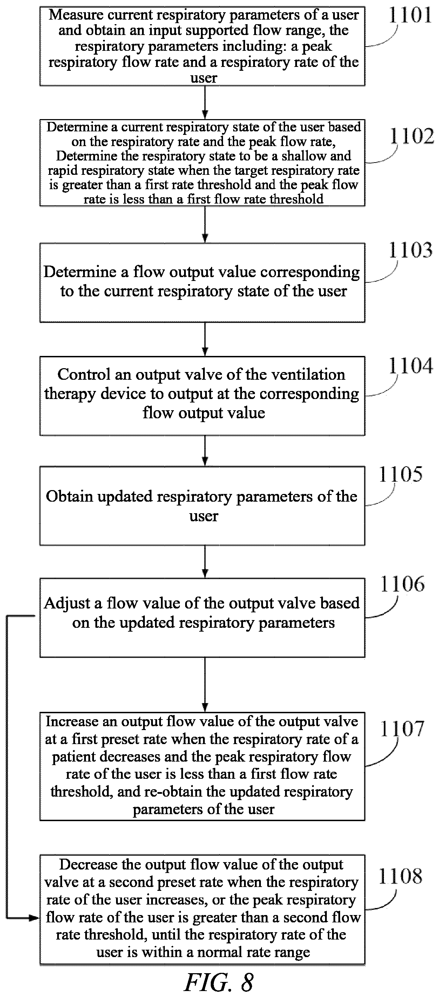

Fig. 8 is another complete flow chart of the steps of a flow adjusting method for a ventilation therapy device according to an embodiment of the present invention. As shown in Fig. 8, the method includes the following steps.

In step 1101, current respiratory parameters of a user are measured and an input supported flow range is obtained. The respiratory parameters include a peak respiratory flow rate and a respiratory rate of the user.

The specific implementation of this step may refer to step 101 described above, and will not be described in detail in this embodiment.

In step 1102, a current respiratory state of the user is determined based on the respiratory rate and the peak flow rate; and when the target respiratory rate is less than the second rate threshold and the peak flow rate is less than the second flow rate threshold and greater than the first flow rate threshold, the respiratory state is determined to be a slow and normal-depth respiratory state.

The slow and normal-depth respiratory state refers to a respiratory state characterized by a normal respiratory depth and a slower respiratory rate, which corresponds to the respiratory parameters including a normal and even peak respiratory flow rate and a slow respiratory rate. Specifically, in an optional implementation provided by an embodiment of the present invention, the corresponding respiratory parameters for determining the slow and normal respiratory state are set as follows: when the target respiratory rate is less than the second rate threshold and the peak flow rate is less than the second flow rate threshold and greater than the first flow rate threshold, i.e., when the respiratory rate is less than 10 Bpm and the peak respiratory flow rate is between 20 L/min and 35 L/min, the current respiratory state of the user is determined to be the slow and normal respiratory state.

In practical applications, the determination criteria for different respiratory states can be adaptively adjusted by the medical personnel according to the actual situations and work experiences, and are not limited in the embodiments of the present invention.

In step 1103, a flow output value corresponding to the current respiratory state of the user is determined.

The specific implementation of this step may refer to step 103 described above, and will not be described in detail in this embodiment.

**Optionally,** step 1103 may specifically include the following sub-step.

In sub-step 11031, a target supported flow value corresponding to the current respiratory state of the user is determined from the supported flow range based on the correspondence between respiratory states and values within the supported flow range, and a flow output value corresponding to the current respiratory state is calculated based on the target supported flow value.

Further, in step 1102, after determining that the current respiratory state of the user is the slow and normal respiratory state, the flow output value corresponding to the slow and normal respiratory state can be matched accordingly. In an embodiment of the present invention, for a user in the slow and normal respiratory state, the corresponding flow output value is composed of the sum of the lower limit value of the preset supported flow range, the baseline flow value and the preset redundant flow value.

In an embodiment of the present invention, the preset supported flow set by the medical personnel is a value range, with an upper limit value and a lower limit value. For example, in an optional implementation, for the flow output values corresponding to the user in the slow and normal respiratory state, the upper limit value of the preset supported flow value is set to 30 L, and the lower limit value is set to 10 L; then, the final corresponding flow output value is specifically: the peak respiratory flow rate of 20 L + the lower limit value of 10 L of the preset supported flow range + the preset redundant flow value of 5 L = 35 L.

In step 1104, the output valve of the ventilation therapy device is controlled to output at the corresponding flow output value.

The specific implementation of this step may refer to step 104 described above, and will not be described in detail in this embodiment.

In step 1105, updated respiratory parameters of the user are obtained.

The specific implementation of this step may refer to step 105 described above, and will not be described in detail in this embodiment.

In step 1106, a flow value of the output valve is adjusted based on the updated respiratory parameters.

The specific implementation of this step may refer to step 106 described above, and will not be described in detail in this embodiment.

In step 1107, when the respiratory rate of the user decreases and the peak respiratory flow rate of the user is less than a first flow rate threshold, an output flow value of the output valve is increased at a first preset rate and re-obtaining the updated respiratory parameters of the user.

After determining the flow output value of the ventilation therapy device as described in step 1103, the ventilation therapy device outputs airflow to the user at this value. Within a certain time, when the respiratory parameters of the user change, the device will adjust the flow output value controlled by the output valve correspondingly based on the changed respiratory parameters. If the respiratory rate of the user decreases and the peak respiratory flow rate of the user is less than the first flow rate threshold, the supported flow of the device is increased correspondingly at the first preset rate, and the updated respiratory parameters of the user are continuously obtained. In an optional implementation provided by an embodiment of the present invention, the first preset rate is specifically set as follows: adjusting the preset supported flow value every 5 minutes at a rate of 10 litres per minute (10 L/min).

In step 1108, when the respiratory rate of the user increases, or the peak respiratory flow rate of the user is greater than a second flow rate threshold, the output flow value of the output valve is decreased at a second preset rate until the respiratory rate of the user is within a normal rate range.

Conversely, when the respiratory rate of the user increases, or the peak respiratory flow rate of the user is greater than the second flow rate threshold, it indicates that the previously set flow output value of the device has taken effect. In this case, the supported flow of the device is decreased at the second preset rate to decrease the output flow value of the output valve. In an optional implementation provided by an embodiment of the present invention, the second preset rate is specifically set as follows: adjusting the preset supported flow value every 30 minutes at a rate of 5 litres per minute (5 L/min). The above steps are then repeatedly performed until the measured respiratory rate tends to be normal, at which point the adjustment can be stopped.

In summary, in the flow adjusting method for a ventilation therapy device according to the embodiments of the present invention, by means of the ventilation therapy device, current respiratory parameters of a user are measured first, the respiratory parameters including a peak respiratory flow rate of the user, and a time interval between two consecutive peak flow rates; a target respiratory rate is calculated based on the time interval between two consecutive peak flow rates, a current respiratory state of the user is determined based on both the respiratory rate and the peak flow rate, and then outputting is performed at a corresponding flow output value based on the current respiratory state of the user; the respiratory parameters of the user are re-obtained after an output valve is controlled according to the flow output value, and a flow value of the output valve is adjusted based on changes in the respiratory parameters; and an output airflow flow value of the device is adjusted in real time by monitoring the respiratory state of the user. Different ventilator output operation schemes are specified for respiratory states of different users, thus solving the problems in the prior art of too uniform results adjusted by a preset algorithm of the device and poor support for the differentiated needs of different users.

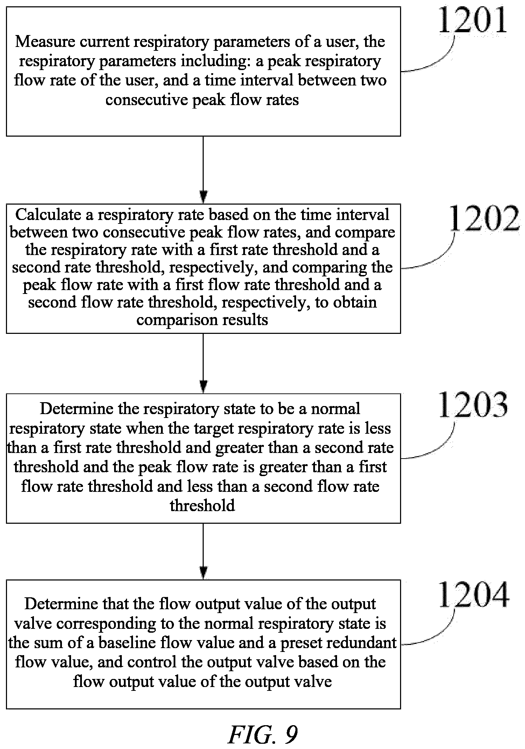

Referring to Fig. 9, Fig. 9 is another complete flow chart of the steps of a flow adjusting method for a ventilation therapy device according to an embodiment of the present invention. As shown in Fig. 9, the method includes the following steps.

In step 1201, current respiratory parameters of a user are measured and an input supported flow range is obtained. The respiratory parameters include a peak respiratory flow rate and a respiratory rate of the user.

The specific implementation of this step may refer to step 101 described above, and will not be described in detail in this embodiment.

In step 1202, a respiratory rate is calculated based on a time interval between two consecutive peak flow rates, and the respiratory rate is compared with a first rate threshold and a second rate threshold, respectively, and the peak flow rate is compared with a first flow rate threshold and a second flow rate threshold, respectively, to obtain comparison results.

The specific implementation of this step may refer to step 1021 described above, and will not be described in detail in this embodiment.

In step 1203, the respiratory state is determined to be a normal respiratory state when the target respiratory rate is less than a first rate threshold and greater than a second rate threshold and the peak flow rate is greater than a first flow rate threshold and less than a second flow rate threshold.

**The normal** respiratory state refers to a respiratory state characterized by a normal respiratory depth and a normal respiratory rate, which corresponds to the respiratory parameters including a normal and even peak respiratory flow rate and a normal and even respiratory rate. Specifically, in an optional implementation provided by an embodiment of the present invention, the corresponding respiratory parameters for determining the normal respiratory state are set as follows: when the target respiratory rate is less than the first rate threshold and greater than the second rate threshold and the peak flow rate is greater than the first flow rate threshold and less than the second flow rate threshold, i.e., when the respiratory rate is between 10 Bpm and 20 Bpm and the peak respiratory flow rate is between 20 L/min and 35 L/min, the current respiratory state of the user is determined to be a slow and normal respiratory state.

**In** practical applications, the determination criteria for different respiratory states can be adaptively adjusted by the medical personnel according to the actual situations and work experiences, and are not limited in the embodiments of the present invention.

**In** step 1204, the flow output value of the output valve corresponding to the normal respiratory state is the sum of a baseline flow value and a preset redundant flow value, and the output valve is controlled based on the flow output value of the output valve.

Further, in step 1203, after determining that the current respiratory state of the user is the normal respiratory state, the flow output value corresponding to the normal respiratory state can be matched accordingly. **In** an embodiment of the present invention, for a user in the normal respiratory state, the corresponding flow output value is composed of the sum of the baseline flow value and the preset redundant flow value.

**In** an embodiment of the present invention, for a user in the normal respiratory state, no additional preset supported flow is needed for assisted respiration supply. The device only needs to meet a minimum inspiratory flow requirement of the user, which corresponds to the horizontal position of the standard output value reference line 203 in Fig. 2. Accordingly, the final corresponding flow output value is specifically: the peak respiratory flow rate of 5 L + the preset redundant flow value of 5 L = 10 L.

Moreover, for a user in the normal respiratory state, each of the respiratory rate and the peak respiratory flow rate in the respiratory parameters is already within a normal range, so there is no need to set corresponding flow output values for subsequent adjustment. In this state, the ventilation therapy device only needs to ensure the basic respiratory needs of the user. It should be noted that for some of the steps in other embodiments listed above, include steps 305-306, steps 405-406, steps 505-506, steps 805-806, steps 905-906, and steps 1105-1106, the measurement of the changed respiratory parameters of the user and the secondary adjustment of the flow output value of the ventilation therapy device are both aimed at achieving the normal respiratory state described in step 1203 as the final adjustment goal.

In summary, in the flow adjusting method for a ventilation therapy device according to the embodiments of the present invention, by means of the ventilation therapy device, current respiratory parameters of a user are measured first, the respiratory parameters including a peak respiratory flow rate of the user, and a time interval between two consecutive peak flow rates; a target respiratory rate is calculated based on the time interval between two consecutive peak flow rates, a current respiratory state of the user is determined based on both the respiratory rate and the peak flow rate, and then outputting is performed at a corresponding flow output value based on the current respiratory state of the user; the respiratory parameters of the user are re-obtained after an output valve is controlled according to the flow output value, and a flow value of the output valve is adjusted based on changes in the respiratory parameters; and an output airflow flow value of the device is adjusted in real time by monitoring the respiratory state of the user. Different ventilator output operation schemes are specified for respiratory states of different users, thus solving the problems in the prior art of too uniform results adjusted by a preset algorithm of the device and poor support for the differentiated needs of different users.

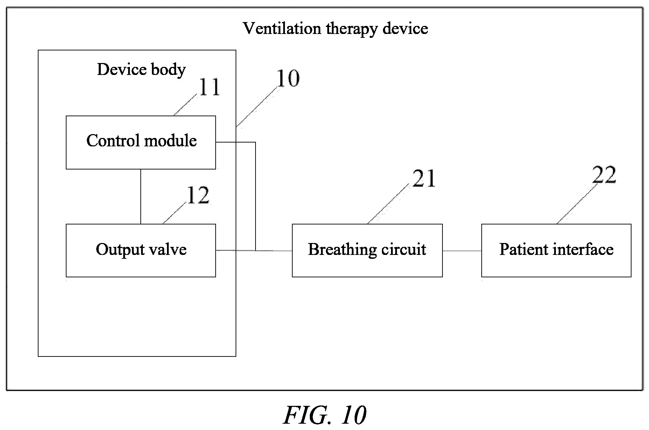

Referring to Fig. 10, Fig. 10 is a schematic diagram showing a composition relationship of functional components of a ventilation therapy device according to an embodiment of the present invention. As shown in Fig. 10, the device includes:
a device body 10, a breathing circuit 21, a user interface 22, an output valve 12 and a control module 11. The device body 10 is configured to output a gas at a preset pressure and a preset flow through an output end; the breathing circuit 21 includes a first end and a second end that are in communication with each other, the first end of the breathing circuit 21 being in communication with the output end of the device body 10, and the second end of the breathing circuit 21 being connected to the user interface 22; and the user interface 22 is configured to be worn at the nasal cavity of a user. When the user interface 22 is worn at the nasal cavity of the user, an exhalation gap is provided between the user interface 22 and the nasal cavity of the user.

The control module 11 is configured to: measure current respiratory parameters of the user, the respiratory parameters including a peak respiratory flow rate of the user and a time interval between two consecutive peak flow rates; calculate a respiratory rate based on the time interval between two consecutive peak flow rates, and determine a current respiratory state of the user based on the respiratory rate and the peak flow rate; determine a flow output value corresponding to the current respiratory state of the user; and after controlling the output valve 12 based on the flow output value, obtain updated respiratory parameters of the user and adjust a flow value of the output valve based on the updated respiratory parameters.

In practical applications, the control module 11 measures the current respiratory parameter of the user by means of the breathing circuit 21 and the user interface 22, determines the current respiratory state of the user based on the respiratory parameters, then controls the output valve 12 based on the current respiratory state of the user to output at a corresponding flow output value, re-obtains the respiratory parameters of the user after controlling the output valve according to the flow output value, and adjusts the flow value of the output 12 according to the changes in the respiratory parameters. Specifically, the ventilation therapy device shown in Fig. 10 is configured to implement the flow adjusting method for a ventilation therapy device as described in any of steps 301 to 306 according to the embodiments.

It should be noted that the ventilation therapy device according to the embodiments of the present invention does not define the specific type of the device. In practical applications, the ventilation therapy device may specifically include a ventilator, an oxygen therapy device, etc.

In summary, in the ventilation therapy device according to the embodiments of the present invention, by means of the ventilation therapy device, current respiratory parameters of a user are measured first, the respiratory parameters including a peak respiratory flow rate of the user, and a time interval between two consecutive peak flow rates; a target respiratory rate is calculated based on the time interval between two consecutive peak flow rates, a current respiratory state of the user is determined based on both the respiratory rate and the peak flow rate, and then outputting is performed at a corresponding flow output value based on the current respiratory state of the user; the respiratory parameters of the user are re-obtained after an output valve is controlled according to the flow output value, and a flow value of the output valve is adjusted based on changes in the respiratory parameters; and an output airflow flow value of the device is adjusted in real time by monitoring the respiratory state of the user. Different ventilator output operation schemes are specified for respiratory states of different users, thus solving the problems in the prior art of too uniform results adjusted by a preset algorithm of the device and poor support for the differentiated needs of different users.

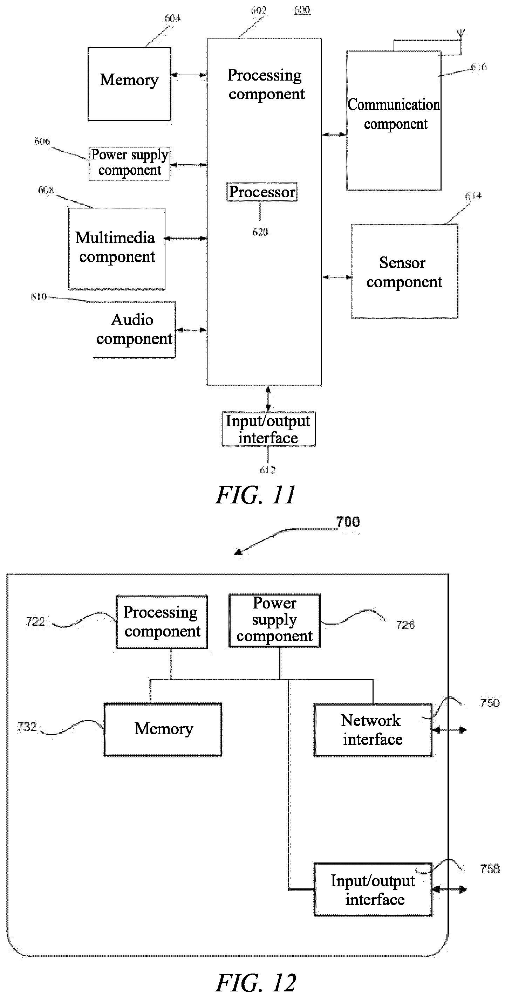

Fig. 11 is a block diagram of an electronic device 600 according to an exemplary embodiment. For example, the electronic device 600 may be a mobile phone, a computer, a digital broadcast terminal, a messaging device, a game console, a tablet device, a medical device, a fitness device, a personal digital assistant, etc.

Referring to Fig. 11, the electronic device 600 may include one or more of the following components: a processing component 602, a memory 604, a power supply component 606, a multimedia component 608, an audio component 610, an input/output (I/O) interface 612, a sensor component 614, and a communication component 616.

The processing component 602 generally controls the overall operation of the electronic device 600, such as operations related to display, telephone calls, data communications, camera operations and recording operations. The processing component 602 may include one or more processors 620 to execute instructions to perform all or some of the steps of the method described above. In addition, the processing component 602 may include one or more modules that facilitate interaction between the processing component 602 and other components. For example, the processing component 602 may include a multimedia module to facilitate interaction between the multimedia component 608 and the processing component 602.

The memory 604 is configured to store various types of data to support operations on the electronic device 600. Examples of such data include instructions for any application or method operating on the electronic device 600, contact data, phone book data, messages, pictures, multimedia, etc. The memory 604 may be implemented by any type of volatile or non-volatile storage devices or a combination thereof, such as static random access memory (SRAM), electrically erasable programmable read-only memory (EEPROM), erasable programmable read-only memory (EPROM), programmable read-only memory (PROM), read-only memory (ROM), magnetic memory, flash memory, magnetic disk or optical disk.

The power supply component 606 provides power to various components of the electronic device 600. The power supply component 606 may include a power management system, one or more power supplies, and other components associated with power generation, management, and distribution for the electronic device 600.

The multimedia component 608 includes a screen that provides an output interface between the electronic device 600 and the user. In some embodiments, the screen may include a liquid crystal display (LCD) and a touch panel (TP). If the screen includes a touch panel, the screen may be implemented as a touch screen to receive input signals from the user. The touch panel includes one or more touch sensors to sense touches, swipes, and gestures on the touch panel. The touch sensor can not only sense a boundary of touch or swipe actions, but can also measure the duration and pressure associated with the touch or swipe actions. In some embodiments, the multimedia component 608 includes a front camera and/or a rear camera. The front camera and/or the rear camera may receive external multimedia data when the electronic device 600 is in an operational mode, such as a shooting mode or a multimedia mode. Each front camera and rear camera may be a fixed optical lens system or have a focal length and optical zoom capabilities.

The audio component 610 is configured to output and/or input audio signals. For example, the audio component 610 includes a microphone (MIC). When the electronic device 600 is in an operational mode, such as a call mode, a recording mode or a voice recognition mode, the microphone is used to receive external audio signals. The received audio signals may be further stored in the memory 604 or transmitted via the communication component 616. In some embodiments, the audio component 610 further includes a speaker for outputting an audio signal.

The I/O interface 612 provides an interface between the processing component 602 and a peripheral interface module, which may be a keyboard, a click wheel, a button, etc. Such a button may include, but not limited to, a Homepage button, a volume button, a start button, and a lock button.

The sensor component 614 includes one or more sensors for providing state assessments of various aspects for the electronic device 600. For example, the sensor component 614 may detect an on/off state of the electronic device 600 and the relative positioning of components. For example, the components include a display and a keypad for the electronic device 600. The sensor component 614 may also detect changes in the position of the electronic device 600 or a component of the electronic device 600, the presence or absence of contact between the user and the electronic device 600, the orientation or acceleration/deceleration of the electronic device 600, and changes in the temperature of the electronic device 600. The sensor component 614 may include a proximity sensor configured to detect the presence of a nearby object in the absence of any physical contact. The sensor component 614 may further include an optical sensor, such as a complementary metal oxide semiconductor (CMOS) or charge-coupled device (CCD) image sensor, for use in imaging applications. In some embodiments, the sensor component 614 may further include an acceleration sensor, a gyroscope sensor, a magnetic sensor, a pressure sensor or a temperature sensor.

The communication component 616 is configured to facilitate wired or wireless communication between the electronic device 600 and other devices. The electronic device 600 can access a wireless network compliant with communication standards, such as WiFi, a carrier network (such as 2G, 3G, 4G, or 5G), or a combination thereof. In an exemplary embodiment, the communication component 616 receives broadcast signals or broadcast-related information from an external broadcast management system via a broadcast channel. In one exemplary embodiment, the communication component 616 further includes a near field communication (NFC) module to facilitate short-range communication. For example, the NFC module may be based on radio frequency identification (RFID) technology, infrared data association (IrDA) technology, ultra-wideband (UWB) technology, Bluetooth (BT) technology and other technologies.

In an exemplary embodiment, the electronic device 600 may be implemented by one or more application specific integrated circuits (ASICs), digital signal processors (DSPs), digital signal processing devices (DSPDs), programmable logic devices (PLDs), field programmable gate arrays (FPGAs), controllers, microcontrollers, microprocessors or other electronic elements for implementing a flow adjusting method for a ventilation therapy device according to the embodiments of the present invention.

In an exemplary embodiment, a non-transitory computer-readable storage medium including instructions is also provided, such as the memory 604 including instructions executable by the processor 620 of the electronic device 600 to accomplish the method described above. For example, the non-transitory storage medium may be a read-only memory (ROM), a random access memory (RAM), an optical disk read-only memory (CD-ROM), a tape, a floppy disk, an optical data storage device, etc.

Fig. 12 is a block diagram of an electronic device 700 according to an exemplary embodiment. For example, the electronic device 700 may be provided as a server. Referring to Fig. 12, the electronic device 700 includes a processing component 722, and further includes one or more processors, and a memory resource represented by a memory 732, which is configured to store instructions executable by the processing component 722, such as an application program. The application program stored in the memory 732 may include one or more modules each corresponding to a set of instructions. In addition, the processing component 722 is configured to execute instructions to perform a flow adjusting method for a ventilation therapy device according to the embodiments of the present invention.

The electronic device 700 may further include a power supply component 726 configured to perform power supply management of the electronic device 700, a wired or wireless network interface 750 configured to connect the electronic device 700 to the Internet, and an input/output (I/O) interface 758. The electronic device 700 may be operated based on an operating system stored in the memory 732, such as Windows Server TM, Mac OS XTM, Unix TM, Linux TM, FreeBSD TM, or the like.

Other implementations of the present invention would readily occur to those skilled in the art after considering the description and the practice of the invention disclosed herein. The present invention is intended to cover any variation, usage or adaptation change of the present invention, which follow the general principles of the present invention and include common general knowledge or customary technical means in the art that are not disclosed in the present disclosure. The description and embodiments are merely to be regarded as exemplary, while the true scope and spirit of the present invention are indicated by the claims below.

It should be understood that the present invention is not limited to the precise structures described above and shown in the accompanying drawings, and various modifications and changes may be made without departing from the scope thereof. The scope of the present invention is merely defined by the appended claims.

## Claims

1. A flow adjusting method for a ventilation therapy device, **characterized by** comprising:
measuring current respiratory parameters of a user and obtaining an input supported flow range, the respiratory parameters comprising a peak respiratory flow rate and a respiratory rate of the user;
determining a current respiratory state of the user based on the respiratory rate and the peak flow rate, the supported flow range being a flow range that is determined based on the current respiratory state of the user and meets respiratory demands of the user;
determining a flow output value corresponding to the current respiratory state of the user based on the supported flow range and the current respiratory state of the user; and
controlling an output valve of the ventilation therapy device to output at the corresponding flow output value.

2. The method according to claim 1, **characterized in that** determining a current respiratory state of the user based on the respiratory rate and the peak flow rate comprises:
comparing the respiratory rate with a first rate threshold and a second rate threshold, respectively, and comparing the peak flow rate with a first flow rate threshold and a second flow rate threshold, respectively, to obtain comparison results, wherein the first rate threshold is greater than the second rate threshold, and the first flow rate threshold is less than the second flow rate threshold; and
determining a current respiratory state of the user based on the comparison results.

3. The method according to claim 1, **characterized in that** determining a corresponding flow output value based on the supported flow range and the current respiratory state of the user comprises:
determining a target supported flow value corresponding to the current respiratory state of the user from the supported flow range based on the correspondence between respiratory states and values within the supported flow range, and calculating a flow output value corresponding to the current respiratory state based on the target supported flow value.

4. The method according to claim 1, **characterized in that** after controlling an output valve of the ventilation therapy device to output at the corresponding flow output value, the method further comprises:
obtaining updated respiratory parameters of the user; and
adjusting a flow value of the output valve based on the updated respiratory parameters.

5. The method according to claim 2, **characterized in that** determining a current respiratory state of the user based on the comparison results comprises:
determining the respiratory state to be a shallow and rapid respiratory state when the target respiratory rate is greater than the first rate threshold and the peak flow rate is less than the first flow rate threshold.

6. The method according to claim 3, **characterized in that** determining a target supported flow value corresponding to the current respiratory state of the user from the supported flow range based on the correspondence between respiratory states and values within the supported flow range, and calculating a flow output value corresponding to the current respiratory state based on the target supported flow value comprises:
when the respiratory state is a shallow and rapid respiratory state, determining a first target supported flow value corresponding to the shallow and rapid respiratory state based on the correspondence between preset respiratory states and supported flow values; and
taking the sum of the first target supported flow value, a baseline flow value and a preset redundant flow value as a flow output value corresponding to the shallow and rapid respiratory state, the baseline flow value being equal to a current peak respiratory flow rate of the user, and the first target supported flow value being a midpoint of the supported flow range.

7. The method according to claim 4, **characterized in that** when the respiratory state is a shallow and rapid respiratory state, adjusting a flow value of the output valve based on the updated respiratory parameters comprises:
increasing an output flow value of the output valve at a first preset rate when the respiratory rate of the user increases or remains unchanged, or the peak respiratory flow rate of the user decreases or remains unchanged, until the first target supported flow value reaches an upper limit value of the supported flow range, or the respiratory rate of the user is within a normal rate range; and
decreasing the output flow value of the output valve at a second preset rate when the respiratory rate of the user decreases, or the peak respiratory flow rate of the user increases, until the respiratory rate of the user is within the normal rate range, or the peak respiratory flow rate of the user is within a normal flow rate range.

8. The method according to claim 2, **characterized in that** determining a current respiratory state of the user based on the comparison results comprises:
determining the respiratory state to be a shallow and slow respiratory state when the target respiratory rate is less than the second rate threshold and the peak flow rate is less than the first flow rate threshold.

9. The method according to claim 3, **characterized in that** determining a target supported flow value corresponding to the current respiratory state of the user from the supported flow range based on the correspondence between respiratory states and values within the supported flow range, and calculating a flow output value corresponding to the current respiratory state based on the target supported flow value comprises:
when the respiratory state is a shallow and slow respiratory state, determining a second target supported flow value corresponding to the shallow and slow respiratory state based on the correspondence between preset respiratory states and supported flow values; and
taking the sum of the second target supported flow value, a baseline flow value and a preset redundant flow value as a flow output value corresponding to the shallow and slow respiratory state, the baseline flow value being equal to a current peak respiratory flow rate of the user, and the second target supported flow value being an upper limit value of the supported flow range.

10. The method according to claim 4, **characterized in that** when the respiratory state is a shallow and slow respiratory state, adjusting a flow value of the output valve based on the updated respiratory parameters comprises:
decreasing an output flow value of the output valve at a second preset rate when the respiratory rate of the user increases, or the peak respiratory flow rate of the user increases, until the respiratory rate of the user is within a normal rate range, or the peak respiratory flow rate of the user is within a normal flow rate range.

11. The method according to claim 2, **characterized in that** determining a current respiratory state of the user based on the comparison results comprises:
determining the respiratory state to be a deep and rapid respiratory state when the target respiratory rate is greater than the first rate threshold and the peak flow rate is greater than the second flow rate threshold.

12. The method according to claim 2, **characterized in that** determining a current respiratory state of the user based on the comparison results comprises:
determining the respiratory state to be a normal-rate deep respiratory state when the target respiratory rate is less than the first rate threshold and greater than the second rate threshold, and the peak flow rate is greater than the second flow rate threshold.

13. The method according to claim 2, **characterized in that** determining a current respiratory state of the user based on the comparison results comprises:
determining the respiratory state to be a normal-rate shallow respiratory state when the target respiratory rate is greater than the second rate threshold and less than the first rate threshold and the peak flow rate is less than the first flow rate threshold.

14. The method according to claim 2, **characterized in that** determining a current respiratory state of the user based on the comparison results comprises:
determining the respiratory state to be a slow and normal-depth respiratory state when the target respiratory rate is less than the second rate threshold and the peak flow rate is greater than the first flow rate threshold and less than the second flow rate threshold.

15. The method according to claim 3, **characterized in that** determining a target supported flow value corresponding to the current respiratory state of the user from the supported flow range based on the correspondence between respiratory states and values within the supported flow range, and calculating a flow output value corresponding to the current respiratory state based on the target supported flow value comprises:
when the respiratory state is any one of a deep and rapid respiratory state, a normal-rate deep respiratory state, a normal-rate shallow respiratory state and a slow and normal-depth respiratory state, determining a third target supported flow value corresponding to the respiratory state based on the correspondence between preset respiratory states and supported flow values; and
taking the sum of the third target supported flow value, a baseline flow value and a preset redundant flow value as a flow output value of the output valve in a target output scheme corresponding to any one of the deep and rapid respiratory state, the normal-rate deep respiratory state and the normal-rate shallow respiratory state, the baseline flow value being equal to a current peak respiratory flow rate of the user, and the third target supported flow value being a lower limit value of the supported flow range.

16. The method according to claim 4, **characterized in that** when the respiratory state is a deep and rapid respiratory state, adjusting a flow value of the output valve based on the updated respiratory parameters comprises:
increasing an output flow value of the output valve at a first preset rate when the respiratory rate of the user increases or remains unchanged, or the peak respiratory flow rate of the user increases, and re-obtaining the updated respiratory parameters of the user; and
decreasing the output flow value of the output valve at a second preset rate when the respiratory rate of the user decreases, or the peak respiratory flow rate of the user decreases, until the respiratory rate of the user is within a normal rate range.

17. The method according to claim 4, **characterized in that** when the respiratory state is a normal-rate deep respiratory state, adjusting a flow value of the output valve based on the updated respiratory parameters comprises:
increasing an output flow value of the output valve at a first preset rate when the respiratory rate of the user is lower than a second rate threshold, or the peak respiratory flow rate of the user increases, until the peak respiratory flow rate of the user is within a normal flow rate range; and
decreasing the output flow value of the output valve at a second preset rate when the peak respiratory flow rate of the user decreases, or the respiratory rate of the user is already within a normal rate range, until the peak respiratory flow rate of the user is within the normal flow rate range.

18. The method according to claim 4, **characterized in that** when the respiratory state is a normal-rate shallow respiratory state, adjusting a flow value of the output valve based on the updated respiratory parameters comprises:
increasing an output flow value of the output valve at a first preset rate when the respiratory rate of the user is less than a second rate threshold, or the peak respiratory flow rate of the user increases, until the peak respiratory flow rate of the user is within a normal flow rate range; and
decreasing the output flow value of the output valve at a second preset rate when the respiratory rate of the user is already within a normal rate range, or the peak respiratory flow rate of the user decreases, until the peak respiratory flow rate of the user is within the normal flow rate range.

19. The method according to claim 4, **characterized in that** when the respiratory state is a slow and normal-depth respiratory state, adjusting a flow value of the output valve based on the updated respiratory parameters comprises:
increasing an output flow value of the output valve at a first preset rate when the respiratory rate of the user decreases and the peak respiratory flow rate of the user is less than a first flow rate threshold, and re-obtaining the updated respiratory parameters of the user; and
decreasing the output flow value of the output valve at a second preset rate when the respiratory rate of the user increases, or the peak respiratory flow rate of the user is greater than a second flow rate threshold, until the respiratory rate of the user is within a normal rate range.

20. The method according to claim 1, **characterized by** further comprising:
comparing the respiratory rate with a first rate threshold and a second rate threshold, respectively, and comparing the peak flow rate with a first flow rate threshold and a second flow rate threshold, respectively, to obtain comparison results, wherein the first rate threshold is greater than the second rate threshold, and the first flow rate threshold is less than the second flow rate threshold;
determining the respiratory state to be a normal respiratory state when the target respiratory rate is less than a first rate threshold and greater than a second rate threshold and the peak flow rate is greater than a first flow rate threshold and less than a second flow rate threshold; and
determining that a flow output value corresponding to the normal respiratory state is the sum of a baseline flow value and a preset redundant flow value, and controlling the output valve based on the flow output value.

21. A ventilation therapy device, **characterized by** comprising a device body, a breathing circuit, a user interface, an output valve, and a control module, wherein
the device body is configured to output gas at a preset pressure and a preset flow rate through an output end, the breathing circuit comprises a first end and a second end that are in communication with each other, and the first end of the breathing circuit is in communication with the output end;
the second end of the breathing circuit is connected to the user interface, and the user interface is configured to be worn at the nasal cavity of a user; when the user interface is worn at the nasal cavity of the user, an exhalation gap is provided between the user interface and the nasal cavity of the user; and
the control module is configured to: measure current respiratory parameters of the user, the respiratory parameters comprising a peak respiratory flow rate of the user and a time interval between two consecutive peak flow rates; calculate a respiratory rate based on the time interval between two consecutive peak flow rates, and determine a current respiratory state of the user based on the respiratory rate and the peak flow rate; determine a flow output value corresponding to the current respiratory state of the user; and after controlling the output valve based on the flow output value, obtain updated respiratory parameters of the user and adjust a flow value of the output valve.

22. An electronic device, **characterized by** comprising: a processor; and
a memory configured to store instructions executable by the processor,
wherein the processor is configured to execute the instructions to implement a method according to any one of claims 1 to 20.

23. A readable storage medium, **characterized in that** instructions in the readable storage medium, when executed by a processor of an electronic device, cause the electronic device to carry out a method according to any one of claims 1 to 20.
